# EUROPEAN PATENT APPLICATION

(11) **EP 4 483 797 A1**
(43) Date of publication of application: **01.01.2025**
(21) Application number: 23181366.8
(22) Date of filing: 26.06.2023
(51) Int. Cl.: A61B 5/0537, A61B 5/145, A61B 5/318, A61B 5/369, A61B 5/389, G16H 50/20

(54) **GLUCOSE LEVEL INFERENCE**

(71) Applicant: Koninklijke Philips N.V., 5656 AG Eindhoven (NL)
(72) Inventor: MUEHLSTEFF, Jens, Eindhoven (NL); PELSSERS, Eduard Gerard Marie, Eindhoven (NL); PAPINI, Gabriele, Eindhoven (NL); COX, Lieke Gertruda Elisabeth, Eindhoven (NL); JOYE, Neil Francis, Eindhoven (NL); DEBETS, Rene Jozef Willem, Eindhoven (NL)
(74) Representative: Philips Intellectual Property & Standards

(57) **Abstract**

Proposed are concepts pertaining to aiding and/or improving glucose level inference. In particular, embodiments of the invention propose obtaining and analysing context information for a monitoring time period during which a biological phenomena of the subject is measured. An adjustment value may be determined for modifying a glucose level inference for the subject (thereby improving inference accuracy by accounting for the measurement context).

## Description

### FIELD OF THE INVENTION

The present invention relates to the field of healthcare, and in particular to the field of determining and monitoring a glucose level of a subject.

### BACKGROUND OF THE INVENTION

Glucose monitoring plays an important role in prevention, management and treatment of diabetes. Different technologies for glucose monitoring have been realized. The most common technique in the home is the finger-sticking method: a small drop of capillary blood is taken from a subject and analysed chemically. However, subcutaneous and continuous blood glucose sensors have also been commercialized. Currently, almost all such sensors are based on enzymatic reactions to measure the concentration of glucose in blood. Testing frequency varies widely from almost never to eight or more times per day.

Recently, in an attempt to avoid the need for blood sampling, alternative glucose measurement modalities using ECG, bioimpedance, and photoplethysmography have been proposed. In such modalities, glucose levels are calculated by mathematical functions using surrogate signal features. However, ECG signals, bio-impedance signals and PPG features are typically sensitive to many conditions, thus limiting the performance/accuracy of such methods.

### SUMMARY OF THE INVENTION

The invention is defined by the claims.

According to examples in accordance with an aspect of the invention, there is provided a method for adjusting inference of a glucose level of a subject. The method comprises obtaining context information describing a physiological property of the subject for a monitoring time period during which a biological phenomena of the subject is measured. The context information is analysed to determine an adjustment value for modifying a glucose level inference for the subject.

Proposed concepts thus aim to provide schemes, solutions, concepts, designs, methods and systems pertaining to aiding and/or improving glucose level inference. In particular, embodiments of the invention propose improvement of the inference of the glucose levels from surrogate measures based on ECG, bioimpedance, photo-plethysmography signals. By obtaining and analysing context information for a monitoring time period during which a biological phenomena of the subject is measured, proposed embodiments may determine an adjustment value for modifying a glucose level inference for the subject (thereby improving inference accuracy by accounting for the measurement context, i.e. the specific context of the subject during which the biological phenomena was measured).

Purely by way of example, proposed embodiments may enable a more accurate glucose level inference method/system to be realized by making use of context information describing a physiological property of the subject. That is, embodiments may take account of measurement conditions that would otherwise negatively impact accuracy of blood glucose level inference.

Embodiments may leverage information from one or more sensors to determine an adjustment for a glucose level inference method/system. The adjustment may, for example, be implemented as a calibration or correction factor for improving the accuracy of a glucose level inference. Embodiments may therefore be used in relation to glucose level determination and/or monitoring so as to support a medical professional when selecting treatment for a subject. Such embodiments may also support clinical planning. Improved Clinical Decision Support (CDS) may therefore be provided by proposed concepts.

Some embodiments may further comprise adjusting a glucose inference process based on the determined adjustment value.

Other embodiments may further comprise: obtaining a biomedical signal describing the measured biological phenomena of the subject; and determining a glucose value for the subject based on the obtained biomedical signal and the adjustment value. Examples of biomedical signals include electrocardiogram (ECG) signals, photoplethysmogram (PPG) signals, and bio-impedance signals, as well as other types of signals such as electroencephalogram (EEG), electromyogram (EMG), and electrooculogram (EOG) signals, Thus, in embodiments, the biomedical signal may comprise at least one of: an ECG signal; a PPG signal; an EEG signal; an EMG signal; an EOG signal; and a bio-impedance signal.

In an embodiment, determining a glucose value may comprise: determining an estimated glucose value based on the obtained biomedical signal; and modifying the estimated glucose value based on the adjustment value to determine a context-specific glucose value for the subject. In this way, the adjustment value may be used to modify (e.g. correct or compensate) an estimated glucose value, rather than modifying a glucose level inference.

In other embodiments, determining a glucose value may comprise: modifying a glucose inference algorithm based on the adjustment value so as to generate a modified inference algorithm; and processing the biomedical signal with the modified inference algorithm to determine the glucose value for the subject. That is, in other implementations, the adjustment value may be used to modify (e.g. correct or compensate) a glucose level inference algorithm so that inferences provided by the algorithm are more accurate (rather than modifying outputs/results after that have been determined by such an algorithm).

In some embodiments, determining a glucose value may comprise: modifying data of the biomedical signal based on the adjustment value so as to generate a modified biomedical data; and determining the glucose value for the subject based on the modified biomedical data. Thus, rather than modifying a glucose level inference algorithm or estimated glucose value (e.g. output from an algorithm), some embodiments may use the adjustment value modify (e.g. correct or compensate) information provided by biomedical signal before it is used/processed for glucose value inference.

The physiological property of the subject may comprise at least one of: posture; temperature; respiration cycle; physical activity; torso position; fluid intake; food intake; sweat rates; blood pressure; heart rhythm; and muscle stress. Various types and/or forms of contextual information may therefore be employed by embodiments in order to enable an adjustment value for improving accuracy of glucose level inference for the subject.

In an embodiment, analysing the context information may comprise: analysing the context information to determine if a predetermined condition is met; responsive to determining that the predetermined condition is not met, determining a zero or null adjustment value; and responsive to determining that the predetermined condition is met, determining a non-zero adjustment value. In this way, a relatively simple, but effective, analysis process may be employed by embodiment, thereby reducing computational resource requirements.

Embodiments may further comprise: generating, based on the adjustment value, a user instruction for guiding the user to perform an action or activity. In this way, embodiments may be used to influence the action(s) and/or activity of a user in a manner which supports more accurate glucose level inference.

According to another aspect, there is provided a computer program product for adjusting inference of a glucose level of a subject, wherein the computer program product comprises a computer-readable storage medium having computer-readable program code embodied therewith, the computer-readable program code configured to perform all of the steps of a proposed embodiment.

Thus, there may also be provided a computer system comprising: a computer program product according to proposed embodiment; and one or more processors adapted to perform a method according to a proposed concept by execution of the computer-readable program code of said computer program product.

According to another aspect, there is provided a system for adjusting inference of a glucose level of a subject, the system comprising: an interface configured to obtain context information describing a physiological property of the subject for a monitoring time period during which a biological phenomena of the subject is measured; and a processor arrangement configured to analyse the context information to determine an adjustment value for modifying a glucose level inference for the subject.

According to yet another aspect, there is provided a glucose monitoring apparatus comprising the system for adjusting inference of a glucose level of a subject according to a proposed embodiment.

The system/apparatus may be remotely located from a user device for assessment of a subject. In this way, a user (such as a medical professional) may have an appropriately arranged system that can receive glucose level data at a location remotely located from the subject. Embodiments may therefore enable a user to perform medical assessment of a subject using a local system (which may, for example, comprise a portable display device, such as a laptop, tablet computer, mobile phone, PDA, etc.). By way of example, embodiments may provide an application for a mobile computing device, and the application may be executed and/or controlled by a user of the mobile computing device.

The apparatus may further include: a server device comprising the interface and processor arrangement; and a client device comprising a user-interface. Dedicated data processing means may therefore be employed for the purpose of adjusting inference of a glucose level of a subject, thus reducing processing requirements or capabilities of other components or devices of the system.

The apparatus may further include a client device, wherein the client device comprises the interface, processor arrangement and a display unit. In other words, a user (such as a doctor or medical professional) may have an appropriately arranged client device (such as a laptop, tablet computer, mobile phone, PDA, etc.) which processes received context information in order to determine an adjustment value for modifying a glucose level inference for the subject and generate a display control signal. Purely by way of example, embodiments may therefore provide a glucose monitoring system that enables monitoring of one or more subjects (e.g. patients) from a single location, wherein real-time communication between a subject and user (e.g. nurse or doctor) is provided and can have its functionality extended or modified according to proposed concepts, for example.

It will be understood that processing capabilities may therefore be distributed throughout the system/apparatus in different ways according to predetermined constraints and/or availability of processing resources.

Proposed embodiments may thus be employed in combination with conventional/existing glucose level measuring and/or monitoring systems. In this way, embodiments may integrate into legacy systems so as to improve and/or extend their functionality and capabilities. An improved glucose monitoring system may therefore be provided by proposed embodiments.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a flow diagram a method for adjusting inference of a glucose level of a subject according to an embodiment of the invention;
Fig. 2 depicts an example of ECG signal amplitude modulation caused by breathing;
Fig. 3 is a simplified block diagram of a glucose monitoring apparatus an exemplary embodiment; and
Fig. 4 is a simplified block diagram of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

Also, it is noted that biomedical signals refer to any measurable biological phenomena that can be detected and recorded using electronic instrumentation. Examples of biomedical signals include electrocardiogram (ECG) signals, photoplethysmogram (PPG) signals, and bio-impedance signals, as well as other types of signals such as electroencephalogram (EEG), electromyogram (EMG), and electrooculogram (EOG) signals.

The invention proposes concepts for aiding and/or improving the inference of a subject's glucose levels from biomedical signals. In particular, embodiments of the invention may provide a method and/or system which obtains and analyses context information for a monitoring time period during which biomedical signals of the subject are detected/measured, and this may determine an adjustment value for modifying a glucose level inference for the subject (thereby improving inference accuracy by accounting for the measurement context).

In particular, proposed concepts may provide an approach to enabling the correction, calibration and/or modification of glucose level inference for a subject based on the measurement context of the subject. Accordingly, embodiments may facilitate more accurate glucose level inference which, in turn, may enable improved or optimal diabetes monitoring and management.

For instance, embodiments may improve the measurement accuracy by considering context boundary conditions for the subject, such as: breathing; posture; physical activity history; muscle stress (e.g. isometric); arm orientation; body location (thorax, upper arm, lower arm, finger, etc); or temperature. Reliable inference may therefore take into account required and essential boundary conditions during the calibration phase and/or during the during the monitoring period.

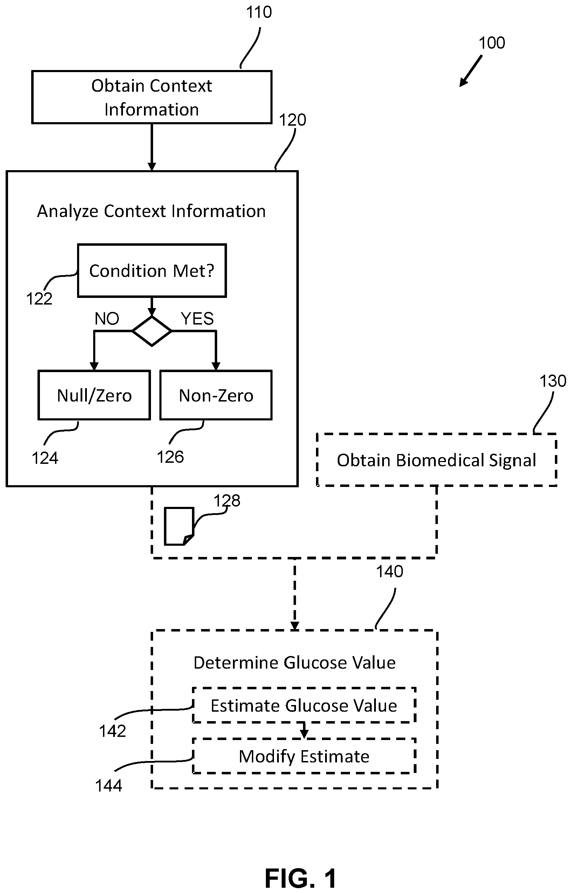

By way of example, a proposed embodiment will now be described with reference to Fig. 1.

Fig. 1 is a flow diagram a method 100 for adjusting inference of a glucose level of a subject according to an embodiment of the invention.

The method begins with step 110 of obtaining context information describing a physiological property of the subject for a monitoring time period during which a biological phenomena of the subject is measured. In this example, the physiological property of the subject comprises at least one of: posture; temperature; respiration cycle; physical activity; torso position; fluid intake; food intake; sweat rates; blood pressure; heart rhythm; and muscle stress. Such information may, for example, be obtained using various measurement systems/arrangements comprising physiological property sensors (e.g., accelerometer, gyroscope, barometer, temperature sensor, camera etc.)

Then, in step 120, the context information is analysed to determine an adjustment value for modifying a glucose level inference for the subject. Here, the step 120 of analysing the context information comprises the sub-steps 122, 124 and 126. Specifically, step 122 comprises analysing the context information to determine if a predetermined condition is met. Responsive to determining (in step 122) that the predetermined condition is not met, the method proceeds to step 124 of determining a zero or null adjustment value. Conversely, responsive to determining (in step 122) that the predetermined condition is met, the method proceeds to step 126 of determining a non-zero adjustment value. As a result of completing step 120, an adjustment value 128 is provided for modifying a glucose level inference for the subject.

The adjustment value 128 may be designed to modify a glucose level inference for the subject in many different ways. For example, it may be for correcting/compensating an obtained biomedical signal of the subject, or it may be for modifying/calibrating a glucose level inference algorithm. In the exemplary embodiment of Fig. 1, however, the adjustment value 128 is for determining a glucose value (via correction/compensation of an estimated glucose value).

More specifically, as depicted by the dashed boxes in Fig. 1, the method further comprises steps 130 and 140.

In step 130, a biomedical signal describing the measured biological phenomena of the subject is obtained. Here, the biomedical signal comprises an ECG signal. However, in other embodiments, the biomedical signal may comprise a PPG signal, an EEG signal, an EMG signal, an EOG signal, or a bio-impedance signal, for example.

In step 140, a glucose value for the subject is determined based on the obtained biomedical signal (from step 13) and the adjustment value 128. Specifically, step 140 of determining a glucose value comprises: (step 142) determining an estimated glucose value based on the obtained biomedical signal; and (step 144) modifying the estimated glucose value based on the adjustment value to determine a context-specific glucose value for the subject. That is, the adjustment value is configured to be employed as a correction/compensation value for modifying the glucose value estimate according to a context of the subject during which the ECG signal was acquired.

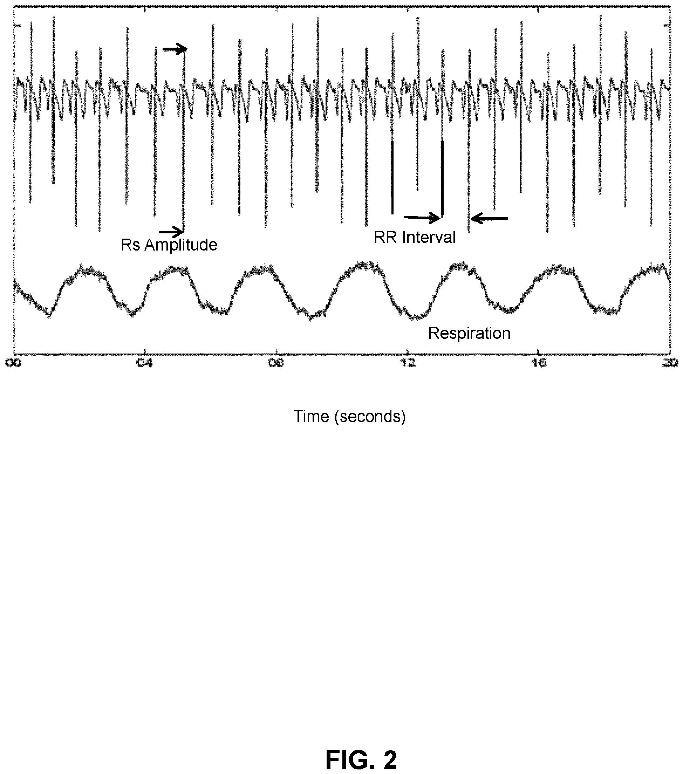

For instance, the heart is not fixated inside the body. It can therefore rotate because of different reasons, e.g. because of breathing and because of posture changes. As a consequence, ECG signal features (such as amplitudes) are dependent on the measurement situation (i.e. measurement context) and boundary conditions. This is demonstrated by the exemplary ECG signal shown in Fig. 2, which shows an example of ECG signal amplitude modulation caused by breathing.

As mentioned above, the adjustment value 128 may be used in other ways to improve glucose level inference (e.g. increase accuracy). For example, instead of including steps 130 and 140, other embodiments may further comprise a step of adjusting a glucose inference process based on the determined adjustment value. In another example, step 140 of determining a glucose value may comprise modifying a glucose inference algorithm based on the adjustment value (include correction factors in the inference (calibration) equation(s)) so as to generate a modified (e.g. more accurate) inference algorithm that accounts for the physiological property of the subject). In yet another example, step 140 of determining a glucose value may comprise modifying data of the biomedical signal based on the adjustment value so as to generate a modified biomedical data (which may then be used to determine a glucose value for the subject).

For instance, embodiments may be employed to objectify / measure required and essential boundary conditions for repeatable and valid measurements during a calibration phase and/or during a glucose level monitoring period. Embodiments may also be employed to check and/or correct glucose inference for repeatable measurement conditions.

Methods according to the proposed concept(s), such as those detailed above, may be implemented in one or more systems. Thus, a system for adjusting inference of a glucose level of a subject may be provided. Further, a glucose monitoring apparatus may incorporate such a system for adjusting inference of a glucose level of a subject. That is, the proposed concept(s) may be integrated into a glucose monitoring apparatus/system so as to provide improved accuracy.

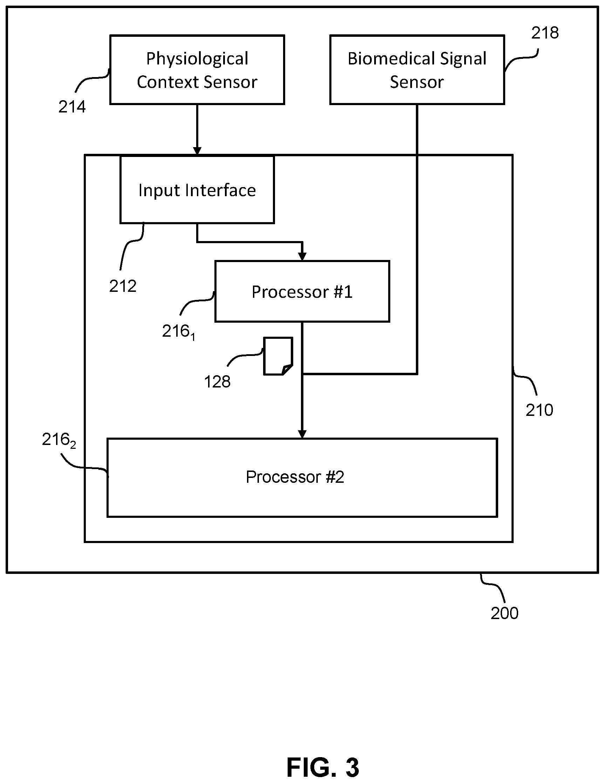

By way of example, a glucose monitoring apparatus according to an embodiment will now be described with reference to Fig. 3.

Fig. 3 is a simplified block diagram of a glucose monitoring apparatus an exemplary embodiment. The glucose monitoring apparatus 200 comprises a system 210 for adjusting inference of a glucose level of a subject according to a proposed concept.

In particular, the system 210 for adjusting inference of a glucose level of a subject comprises an interface 212 that is configured to obtaining context information describing a physiological property of the subject for a monitoring time period during which a biological phenomena of the subject is measured. Here, the interface 212 is configured to receive context information from a physiological context sensor 214 (e.g. accelerometer, gyroscope, barometer, temperature sensor, camera etc.) of the glucose monitoring apparatus 200.

The system 210 for adjusting inference of a glucose level of a subject also comprises a processor arrangement comprising first 216₁ and second 216₂ processors. The first processor 216₁ is configured to analyse the context information to determine an adjustment value 128 for modifying a glucose level inference for the subject. The second processor 216₂ is configured to adjust a glucose inference process of the glucose monitoring apparatus 200 based on the determined adjustment value 128. More specifically, the second processor 216₂ of the processor arrangement is configured to: receive a biomedical signal (e.g. ECG signal, PPG signal, EEG signal, EMG signal, EOG signal, or bio-impedance signal.) from a biomedical signal sensor 218; and determine a glucose value for the subject based on the obtained biomedical signal and the compensation adjustment value 128.

Purely by way of further example, a summary of the components of a glucose level measurement system according to an embodiment may be as follows:
- One or more sensors for acquiring ECG, and/or PPG, and/or bioimpedance signals via a wearable/unobtrusive device (e.g., smartwatch, smart-patch, smart weight scale).
- Motion sensors (e.g., accelerometers, gyroscopes, barometers) to check for appropriate measurement boundary conditions such as: posture and patient activity levels; gyroscopes; barometers; temperature sensors; camera, etc.
- Calibration system (i.e. a system for adjusting inference of a glucose level of a subject) configured to calibrate the glucose estimation algorithm glucose level measurement system. Includes: an interface and storage unit to input glucose reference measurement based on sweat or blood analysis; sensors/devices to assess the calibration conditions (e.g., muscle strength, posture, skin temperature, physical activity history; an EMG or strain gauge, accelerometer(s) or barometer(s), contact or infrared temperature sensor, fitness tracker or smartwatch).
- Means to enforce the calibration condition on future measurements; Sensors/devices to assess the calibration conditions. Includes a feedback system to indicate to the user if the initial calibration conditions are met. May also include a feedback system to guide the user in meeting the initial calibration conditions, e.g. request a user to perform specific procedures quantifying sensitivities of surrogate measures.

The system may also comprise means to acquire additional calibration conditions if initial calibration conditions are not met and adjust the glucose estimation algorithm.

Such a system according to an embodiment, may implement/support the following methods:
- Signal acquisition - During calibration; during the monitoring phase; during a requested specific procedure to check for surrogate sensitivities at glucose levels.
- Trigger a glucose calibration measurement in case of an unknow unsure measurement condition.
- Provide an uncertainty measure giving feedback to the user of the validity of an inferred glucose level.
- Regression functions based on acquired data including cross - sensitivities to boundary condition.
- Corrections in glucose inference equations for example for the ECG amplitude (ECG amp).

By way of further example, pseudo code for a method of adjusting inference of a glucose level of a subject (using a glucose monitoring apparatus) according to an embodiment is as follows:
- IF First use of the device, THEN perform initial calibration: (i) prompt the use of other blood glucose tests; (ii) record the blood glucose value; (iii) record signals used for blood glucose estimation and to track acquisition conditions; (iv) calibrate the glucose estimation algorithm.
- ELSE continue with glucose estimation procedure
- Record signals used for blood glucose estimation (e.g., ECG, PPG, or bioimpedance)
- Record acquisition conditions (e.g., posture, activity, temperature, ...)
- Check the history and characteristics of all the signals/parameters collected by the (wearable/nearable/portable) device.
- IF data matches initial calibration condition and there are no factors influencing the blood glucose estimation, THEN provide the estimation.
- ELSE IF data do not match the initial calibration condition or there are confounding factors.
   - IF confounding factors cannot be corrected (e.g., low quality data or not enough contextual information) THEN (i) do not provide the estimation; (ii) prompt the use of reference blood glucose tests.
   - ELSE IF confounding factors can be corrected or only data different from calibration conditions, THEN (i) do not provide the estimation; (ii) prompt the use of other blood glucose tests (iii) record the blood glucose value and boundary conditions (iv) update calibration and the estimation algorithm (e.g., create several context-tuned blood glucose models).

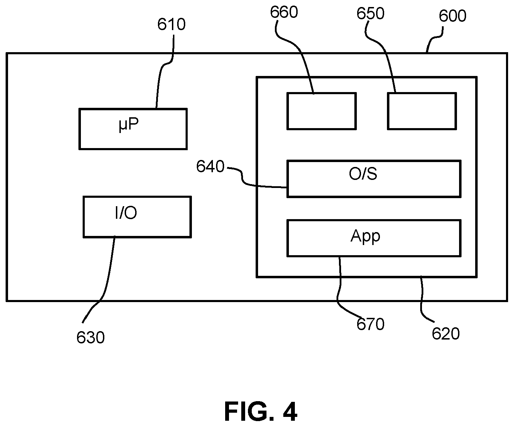

Fig. 4 illustrates an example of a computer 600 within which one or more parts of an embodiment may be employed. Various operations discussed above may utilize the capabilities of the computer 600. For example, one or more parts of the proposed A system for adjusting inference of a glucose level of a subject may be incorporated in any element, module, application, and/or component discussed herein. In this regard, it is to be understood that system functional blocks can run on a single computer or may be distributed over several computers and locations (e.g. connected via internet).

The computer 600 includes, but is not limited to, PCs, workstations, laptops, PDAs, palm devices, servers, storages, and the like. Generally, in terms of hardware architecture, the computer 600 may include one or more processors 610, memory 620, and one or more I/O devices 670 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 610 is a hardware device for executing software that can be stored in the memory 620. The processor 610 can be virtually any custom made or commercially available processor, a central processing unit (CPU), a digital signal processor (DSP), or an auxiliary processor among several processors associated with the computer 600, and the processor 610 may be a semiconductor-based microprocessor (in the form of a microchip) or a microprocessor.

The memory 620 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 620 may incorporate electronic, magnetic, optical, and/or other types of storage media. Note that the memory 620 can have a distributed architecture, where various components are situated remote from one another, but can be accessed by the processor 610.

The software in the memory 620 may include one or more separate programs, each of which comprises an ordered listing of executable instructions for implementing logical functions. The software in the memory 620 includes a suitable operating system (O/S) 650, compiler 640, source code 630, and one or more applications 660 in accordance with exemplary embodiments. As illustrated, the application 660 comprises numerous functional components for implementing the features and operations of the exemplary embodiments. The application 660 of the computer 600 may represent various applications, computational units, logic, functional units, processes, operations, virtual entities, and/or modules in accordance with exemplary embodiments, but the application 660 is not meant to be a limitation.

The operating system 650 controls the execution of other computer programs, and provides scheduling, input-output control, file and data management, memory management, and communication control and related services. It is contemplated by the inventors that the application 660 for implementing exemplary embodiments may be applicable on all commercially available operating systems.

Application 660 may be a source program, executable program (object code), script, or any other entity comprising a set of instructions to be performed. When a source program, then the program is usually translated via a compiler (such as the compiler 640), assembler, interpreter, or the like, which may or may not be included within the memory 620, so as to operate properly in connection with the O/S 650. Furthermore, the application 660 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 670 may include input devices such as, for example but not limited to, a mouse, keyboard, scanner, microphone, camera, etc. Furthermore, the I/O devices 670 may also include output devices, for example but not limited to a printer, display, etc. Finally, the I/O devices 670 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 670 also include components for communicating over various networks, such as the Internet or intranet.

If the computer 600 is a PC, workstation, intelligent device or the like, the software in the memory 620 may further include a basic input output system (BIOS) (omitted for simplicity). The BIOS is a set of essential software routines that initialize and test hardware at startup, start the O/S 650, and support the transfer of data among the hardware devices. The BIOS is stored in some type of read-only-memory, such as ROM, PROM, EPROM, EEPROM or the like, so that the BIOS can be executed when the computer 600 is activated.

When the computer 600 is in operation, the processor 610 is configured to execute software stored within the memory 620, to communicate data to and from the memory 620, and to generally control operations of the computer 600 pursuant to the software. The application 660 and the O/S 650 are read, in whole or in part, by the processor 610, perhaps buffered within the processor 610, and then executed.

When the application 660 is implemented in software it should be noted that the application 660 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 660 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The system of Fig. 3, and the method of Fig. 1, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out a method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

Data describing an adjustment value determined according to an embodiment may be stored on a storage medium. A glucose level value determined according to an embodiment may be stored on the same storage medium or a different storage medium. Such data may be transmitted as a signal modulated onto an electromagnetic carrier wave. The signal may be defined according to a standard for digital communications. The carrier wave may be an optical carrier, a radio-frequency wave, a millimeter wave, or a near field communications wave. It may be wired or wireless.

To the extent that an embodiment is implemented partly or wholly in hardware, the blocks shown in the Figures may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method (100) for adjusting inference of a glucose level of a subject, the method comprising:
obtaining (110) context information describing a physiological property of the subject for a monitoring time period during which a biological phenomena of the subject is measured; and
analysing (120) the context information to determine an adjustment value (128) for modifying a glucose level inference for the subject.

2. The method of claim 1 further comprising: adjusting a glucose inference process based on the determined adjustment value.

3. The method of claim 1 or 2, further comprising:
obtaining (130) a biomedical signal describing the measured biological phenomena of the subject; and
determining (140) a glucose value for the subject based on the obtained biomedical signal and the adjustment value.

4. The method of claim 3, wherein the biomedical signal comprises at least one of: an ECG signal; a PPG signal; an EEG signal; an EMG signal; an EOG signal; and a bio-impedance signal.

5. The method of claim 3 or 4, wherein determining (140) a glucose value comprises:
determining (142) an estimated glucose value based on the obtained biomedical signal; and
modifying (144) the estimated glucose value based on the adjustment value to determine a context-specific glucose value for the subject.

6. The method of claim 3 or 4, wherein determining (140) a glucose value comprises:
modifying a glucose inference algorithm based on the adjustment value so as to generate a modified inference algorithm; and
processing the biomedical signal with the modified inference algorithm to determine the glucose value for the subject.

7. The method of claim 3 or 4, wherein determining (140) a glucose value comprises:
modifying data of the biomedical signal based on the adjustment value so as to generate a modified biomedical data; and
determining the glucose value for the subject based on the modified biomedical data.

8. The method of any of claims 1 to 7, wherein the physiological property of the subject comprises at least one of: posture; temperature; respiration cycle; physical activity; torso position; fluid intake; food intake; sweat rates; blood pressure; heart rhythm; and muscle stress

9. The method of any of claims 1 to 8, wherein analysing the context information comprises:
analysing the context information to determine if a predetermined condition is met;
responsive to determining that the predetermined condition is not met, determining a zero or null adjustment value; and
responsive to determining that the predetermined condition is met, determining a non-zero adjustment value.

10. The method of any of claims 1 to 9, further comprising:
generating, based on the adjustment value, a user instruction for guiding the user to perform an action or activity.

11. A computer program comprising computer program code means adapted, when said computer program is run on a computer, to implement the method of any of claims 1 to 10.

12. A system (210) for adjusting inference of a glucose level of a subject, the system comprising:
an interface (212) configured to obtain context information describing a physiological property of the subject for a monitoring time period during which a biological phenomena of the subject is measured; and
a processor (216) arrangement configured to analyse the context information to determine an adjustment value for modifying a glucose level inference for the subject.

13. The system of claim 12, wherein the processor arrangement is further configured to adjust a glucose inference process based on the determined adjustment value.

14. The system of claim 12 or 13, wherein the processor arrangement is further configured to:
obtain a biomedical signal describing the measured biological phenomena of the subject; and
determine a glucose value for the subject based on the obtained biomedical signal and the compensation adjustment value.

15. Glucose monitoring apparatus (200) comprising the system for adjusting inference of a glucose level of a subject according to claim 12, 13 or 14.
